# EUROPÄISCHE PATENTSCHRIFT

(11) **EP 0 758 324 B1**
(45) Veröffentlichungstag und Bekanntmachung des Hinweises auf die Patenterteilung: **16.03.2005**
(21) Anmeldenummer: 95917347.7
(22) Anmeldetag: 21.04.1995
(51) Int. Cl.: C07D 249/10, A01N 43/00, C07D 471/04, C07D 239/54, C07D 239/56, C07D 207/452, C07D 209/48, C07D 211/88, C07D 231/56, C07D 487/04

(54) **SUBSTITUIERTE AROMATISCHE THIOCARBONSÄUREAMIDE UND IHRE VERWENDUNG ALS HERBIZIDE**
SUBSTITUTED AROMATIC THIOCARBOXYLIC ACID AMIDES AND THEIR USE AS HERBICIDES
AMIDES AROMATIQUES SUBSTITUES D'ACIDES THIOCARBOXYLIQUES ET LEUR UTILISATION COMME HERBICIDES

(30) Priorität: 04.05.1994 DE 4415655; 10.01.1995 DE 19500439
(43) Veröffentlichungstag der Anmeldung: 19.02.1997
(73) Patentinhaber: Bayer CropScience AG, 40789 Monheim (DE)
(72) Erfinder: LINKER, Karl-Heinz, D-51377 Leverkusen (DE); FINDEISEN, Kurt, D-51375 Leverkusen (DE); ANDREE, Roland, D-40764 Langenfeld (DE); DREWES, Mark-Wilhelm, D-40764 Langenfeld (DE); LENDER, Andreas, D-21376 Salzhausen (DE); SCHALLNER, Otto, D-40789 Monheim (DE); HAAS, Wilhelm, D-50259 Pulheim (DE); SANTEL, Hans-Joachim, D-51371 Leverkusen (DE); DOLLINGER, Markus, D-51381 Leverkusen (DE)
(86) Internationale Anmeldenummer: PCT/EP1995/001507
(87) Internationale Veröffentlichungsnummer: WO 1995/030661

(56) Entgegenhaltungen:
- EP-A- 0 052 442
- EP-A- 0 370 332
- EP-A- 0 384 362
- EP-A- 0 537 980
- EP-A- 0 542 363
- WO-A-90/09381
- WO-A-93/14077
- WO-A-93/22303
- WO-A-93/24472
- DE-A- 2 162 439
- DE-A- 2 204 767
- GB-A- 1 080 246
- GB-A- 1 215 858
- US-A- 3 338 913
- US-A- 4 112 095
- US-A- 4 515 791
- US-A- 5 204 352
- CHEMICAL ABSTRACTS, vol. 114, no. 1, 7. Januar 1991, Columbus, Ohio, US; abstract no. 6530s, Seite 646 ; & JP,A,02 193 994 (KYOWA HAKKO KOGYO CO., LTD.) & CHEM. ABS. 12TH COLL. IND. CHEM. SUBST. Seite 10864CS
- JOURNAL OF HETEROCYCLIC CHEMISTRY, Bd.25, Nr.1, Januar 1988, PROVOH US Seiten 129 - 137 R.J. SUNDBERG ET AL. 'Preparation of 2-aryl and 2-aryloxymethyl imidazo[1,2-a]pyridines and related compounds'
- JOURNAL OF MEDICINAL CHEMISTRY, Bd.30, Nr.6, Juni 1987, WASHINGTON US Seiten 1023 - 1029 I. SIRCAR ET AL. 'Cardiotonic agents. 5. 1,2-Dihydro-5[4-(1H-imidazol-1-yl)phenyl]- 6-methyl-2-oxo-3-pyridinecarbonitriles and related compounds. Synthesis and inotropic activity'
- CHEMICAL ABSTRACTS, vol. 103, no. 9, 2. September 1985, Columbus, Ohio, US; abstract no. 71307m, Seite 652 ; & JP,A,59 225 172 (YAMANOUCHI PHARMACEUTICAL CO., LTD.) & CHEM. ABS. 11TH COLL. IND. CHEM. SUBST. Seite 7859CS
- CHEMICAL ABSTRACTS, vol. 91, no. 22, 26. November 1979, Columbus, Ohio, US; abstract no. 184919q, Seite 646-647 ; & JP,A,79 067 419 (KONISHIROKU PHOTO INDUSTRY CO., LTD.)

## Beschreibung

Die Erfindung betrifft neue substituierte aromatische Thiocarbonsäureamide, Verfahren zu ihrer Herstellung und ihre Verwendung als Herbizide.

Es ist bereits bekannt, dass bestimmte aromatische Carbothioamide, wie z.B. 2,6-Dichlor-benzothioamid ("Chlorthiamid") herbizide Eigenschaften aufweisen (vgl. GB-PS 987 253). In der EP-A 0 370 332 werden herbizid wirksame N-Aryl-Stickstoffheterocyclen beschrieben, die sich von den erfindungsgemäßen Verbindungen dadurch unterscheiden, dass sie anstelle des Thiocarbonsäureamid-Restes einen Cyano-Rest besitzen. Die Wirksamkeit dieser vorbekannten Verbindung ist jedoch, insbesondere bei niedrigen Aufwandmengen und Konzentrationen nicht in allen Anwendungsgebieten völlig zufriedenstellend.

Es wurden nun die neuen substituierten aromatischen Thiocarbonsäureamide der allgemeinen Formel (I) gefunden, 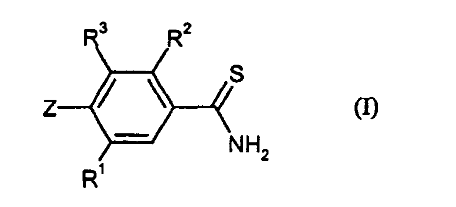 in welcher
- R¹: für Wasserstoff, Fluor, Chlor oder Brom steht,
- R²: für die nachstehende Gruppierung steht,

-A¹-A³
- A¹: für Sauerstoff, Schwefel oder die Gruppierung -N-A⁴- steht, worin A⁴ für Wasserstoff, C₁-C₄-Alkyl, C₁-C₄-Alkoxy oder C₁-C₄-Alkylsulfonyl steht,
- A³: für Wasserstoff, Hydroxy, Amino, Cyano, Isocyanato, Thiocyanato, Nitro, Carboxy, Carbamoyl, Thiocarbamoyl, Sulfo, Chlorsulfonyl, Halogen, für jeweils gegebenenfalls durch Halogen oder C₁-C₄-Alkoxy substituiertes Alkyl, Alkoxy, Alkylthio, Alkylsulfinyl, Alkylsulfonyl, Alkylamino, Dialkylamino, Alkoxycarbonyl oder Dialkoxy(thio)phosphoryl mit jeweils 1 bis 6 Kohlenstoffatomen in den Alkylgruppen, für jeweils gegebenenfalls durch Halogen substituiertes Alkenyl, Alkenyloxy, Alkenylamino, Alkylidenamino, Alkenyloxycarbonyl, Alkinyl, Alkinyloxy, Alkinylamino oder Alkinyloxycarbonyl mit jeweils 2 bis 6 Kohlenstoffatomen in den Alkenyl-, Alkyliden- oder Alkinylgruppen, für jeweils gegebenenfalls durch Halogen, Cyano, Carboxy, C₁-C₄-Alkyl und/oder C₁-C₄-Alkoxycarbonyl substituiertes Cycloalkyl, Cycloalkyloxy, Cycloalkylalkyl, Cycloalkylalkoxy, Cycloalkylidenamino, Cycloalkyloxycarbonyl oder Cycloalkylalkoxycarbonyl mit jeweils 3 bis 6 Kohlenstoffatomen in den Cycloalkylgruppen und gegebenenfalls 1 bis 4 Kohlenstoffatomen in den Alkylgruppen, oder für jeweils gegebenenfalls durch Nitro, Cyano, Carboxy, Halogen, C₁-C₄-Alkyl, C₁-C₄-Halogenalkyl, C₁-C₄-Alkyloxy, C₁-C₄-Halogenalkyloxy und/oder C₁-C₄-Alkoxycarbonyl substituiertes Phenyl, Phenyloxy, Phenyl-C₁-C₄-alkyl, Phenyl-C₁-C₄-alkoxy, Phenyloxycarbonyl oder Phenyl-C₁-C₄-alkoxycarbonyl, (jeweils gegebenenfalls ganz oder teilweise hydriertes) Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolyl-C₁-C₄-alkyl, Furyl-C₁-C₄-alkyl, Thienyl-C₁-C₄-alkyl, Oxazolyl-C₁-C₄-alkyl, Isoxazol-C₁-C₄-alkyl, Thiazol-C₁-C₄-alkyl, Pyridinyl-C₁-C₄-alkyl, Isoxazol-C₁-C₄-alkyl, Thiazol-C₁-C₄-alkyl, Pyridinyl-C₁-C₄-alkyl, Pyrimidinyl-C₁-C₄-alkyl, Pyrazolylmethoxy, Furylmethoxy, für Perhydropyranylmethoxy oder Pyridyl-methoxy steht,
- R³: für Wasserstoff, Fluor, Chlor oder Brom steht, und
- Z: für die nachstehend aufgeführte heterocyclische Gruppierung steht, 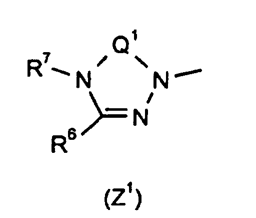
wobei
- Q¹: für -CO- oder -CS- steht,
- R⁶: für Difluormethyl oder Trifluormethyl steht, und
- R⁷: für Methyl, Ethyl, n- oder i-Propyl oder Difluormethyl steht.

Man erhält die neuen substituierten aromatischen Thiocarbonsäureamide der allgemeinen Formel (I), wenn man substituierte aromatische Nitrile der allgemeinen Formel (II), 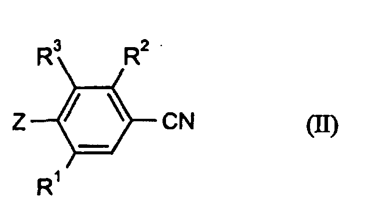 in welcher
- R¹, R², R³ und Z: die oben angegebenen Bedeutungen haben,
mit Schwefelwasserstoff (Hydrogensulfid, H₂S) oder mit Thioacetamid gegebenenfalls in Gegenwart eines Reaktionshilfsmittels und gegebenenfalls in Gegenwart eines Verdünnungsmittels umsetzt.

Die neuen substituierten aromatischen Thiocarbonsäureamide der allgemeinen Formel (I) zeichnen sich durch starke und selektive herbizide Wirksamkeit aus.

In den Definitionen sind die gesättigten oder ungesättigten Kohlenwasserstoffketten, wie Alkyl, Alkandiyl, Alkenyl oder Alkinyl - auch in Verbindung mit Heteroatomen, wie in Alkoxy, Alkylthio oder Alkylamino - jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Gegenstand der Erfindung sind vorzugsweise Verbindungen der Formel (I), in welcher
- R¹: für Wasserstoff, Fluor oder Chlor steht,
- R²: für die nachstehende Gruppierung steht,

-A¹-A³
in welcher
- A¹: für Sauerstoff, Schwefel oder die Gruppierung -N-A⁴- steht, worin A⁴ für Wasserstoff, Methyl, Ethyl, n- oder i-Propyl, Methoxy, Ethoxy, n- oder i-Propoxy, Methylsulfonyl oder Ethylsulfonyl steht,
- A³: für Wasserstoff, Hydroxy, Amino, Cyano, Nitro, Carboxy, Carbamoyl, Sulfo, Fluor, Chlor, Brom, für jeweils gegebenenfalls durch Fluor, Chlor, Methoxy oder Ethoxy substituiertes Methyl, Ethyl, n- oder i-Propyl, n-, i-, s- oder t-Butyl, n-, i-, s- oder t-Pentyl, Methoxy, Ethoxy, n- oder i-Propoxy, n-, i-, s-oder t-Butoxy, n-, i-, s- oder t-Pentyloxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Methoxycarbonyl, Ethoxycarbonyl, n-oder i-Propoxycarbonyl, Dimethoxyphosphoryl, Diethoxyphosphoryl, Dipropoxyphosphoryl oder Diisopropoxyphosphoryl, für jeweils gegebenenfalls durch Fluor oder Chlor substituiertes Propenyl, Butenyl, Propenyloxy, Butenyloxy, Propenylamino, Butenylamino, Propylidenamino, Butyliden-amino, Propenyloxycarbonyl, Butenyloxycarbonyl, Propinyl, Butinyl, Propinyloxy, Butinyloxy, Propinylamino, Butinylamino, Propinyloxycarbonyl oder Butinyloxycarbonyl, für jeweils gegebenenfalls durch Fluor, Chlor, Cyano, Carboxy, Methyl, Ethyl, n- oder i-Propyl, Methoxycarbonyl oder Ethoxycarbonyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopentylidenamino, Cyclohexylidenamino, Cyclopentyloxycarbonyl, Cyclohexyloxycarbonyl, Cyclopentylmethoxycarbonyl oder Cyclohexylmethoxycarbonyl, oder für jeweils gegebenenfalls durch Nitro, Cyano, Carboxy, Fluor, Chlor, Brom, Methyl, Ethyl, n- oder i-Propyl, Trifluormethyl, Methoxy, Ethoxy, n- oder i-Propoxy, Difluormethoxy, Trifluormethoxy, Methoxycarbonyl und/oder Ethoxycarbonyl substituiertes Phenyl, Phenyloxy, Benzyl, Phenylethyl, Benzyloxy, Phenyloxycarbonyl, Benzyloxycarbonyl, (jeweils gegebenenfalls ganz oder teilweise hydriertes) Pyrrolyl, Pyrazolyl, imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolylmethyl, Furylmethyl, Thienylmethyl, Oxazolylmethyl, Isoxazolmethyl, Thiazolmethyl, Pyridinylmethyl, Pyrimidinylmethyl, Pyrazolylmethoxy, Furylmethoxy oder Pyridylmethoxy steht,
- R³: für Wasserstoff, Fluor oder Chlor steht.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte. Diese Restedefinitionen können untereinander, also auch zwischen den angegebenen Bereichen bevorzugter Verbindungen, beliebig kombiniert werden.

Beispiele für die erfindungsgemäßen Verbindungen der Formel (I) sind in den nachstehenden Gruppen aufgeführt. 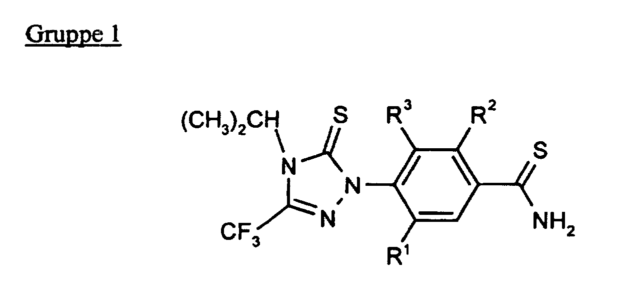
R¹, R² und R³ haben dabei die in der nachstehenden Auflistung angegebenen Bedeutungen:

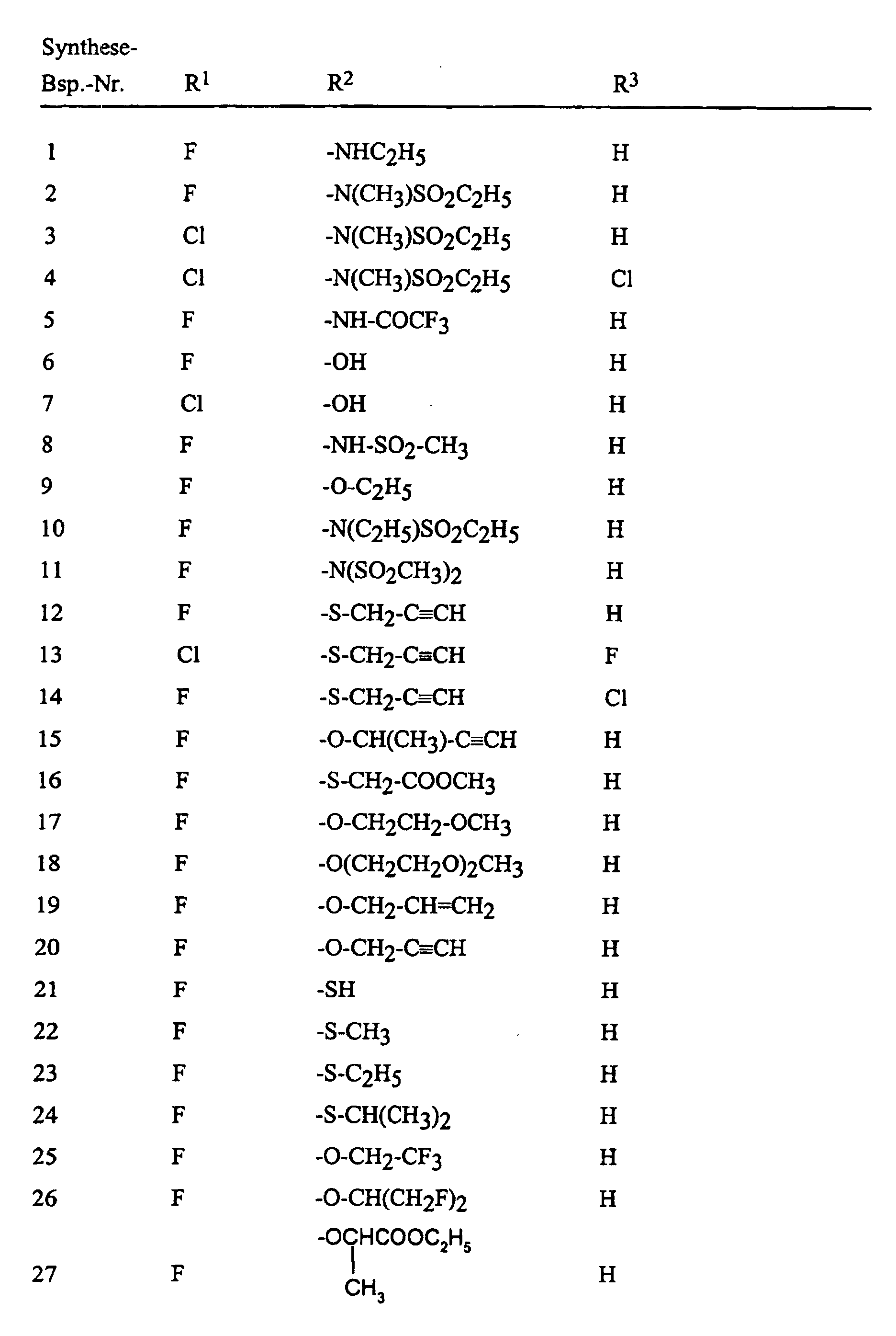

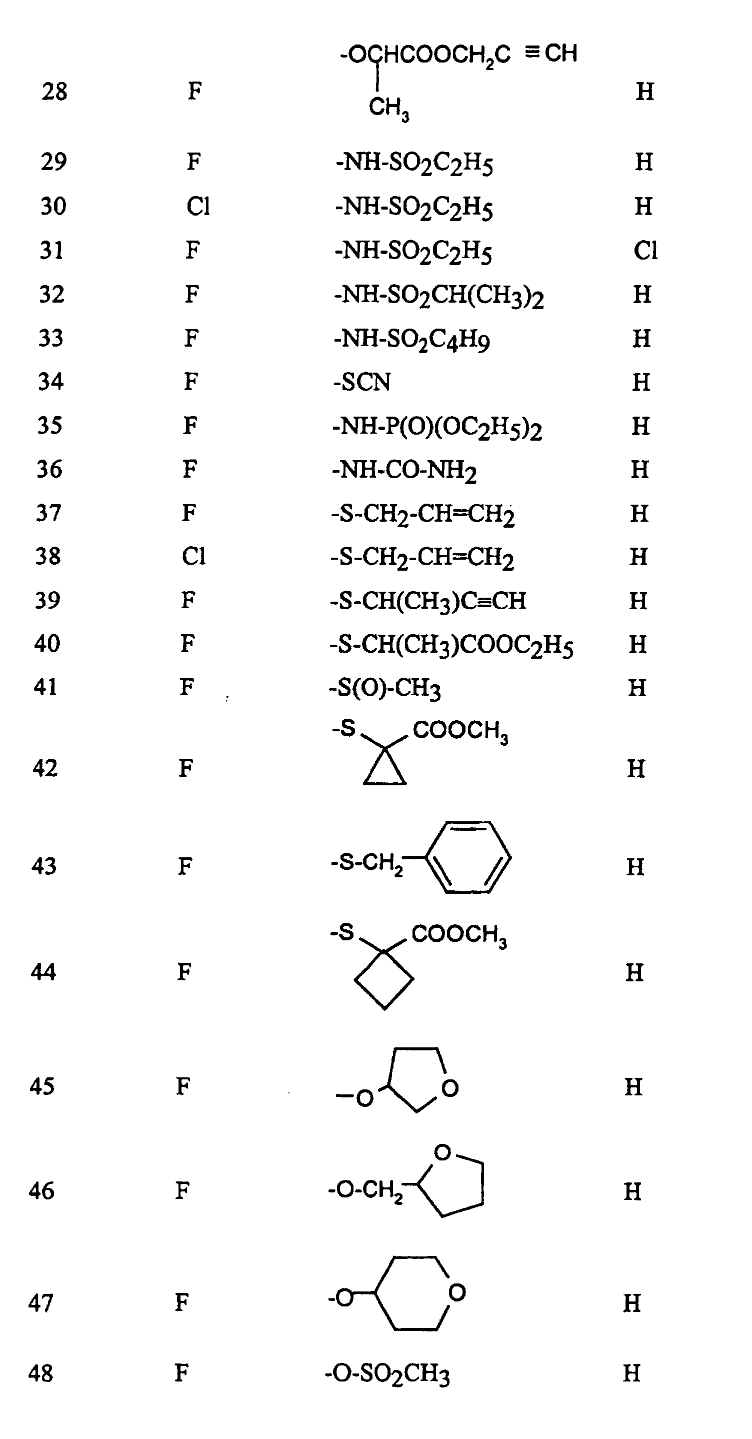

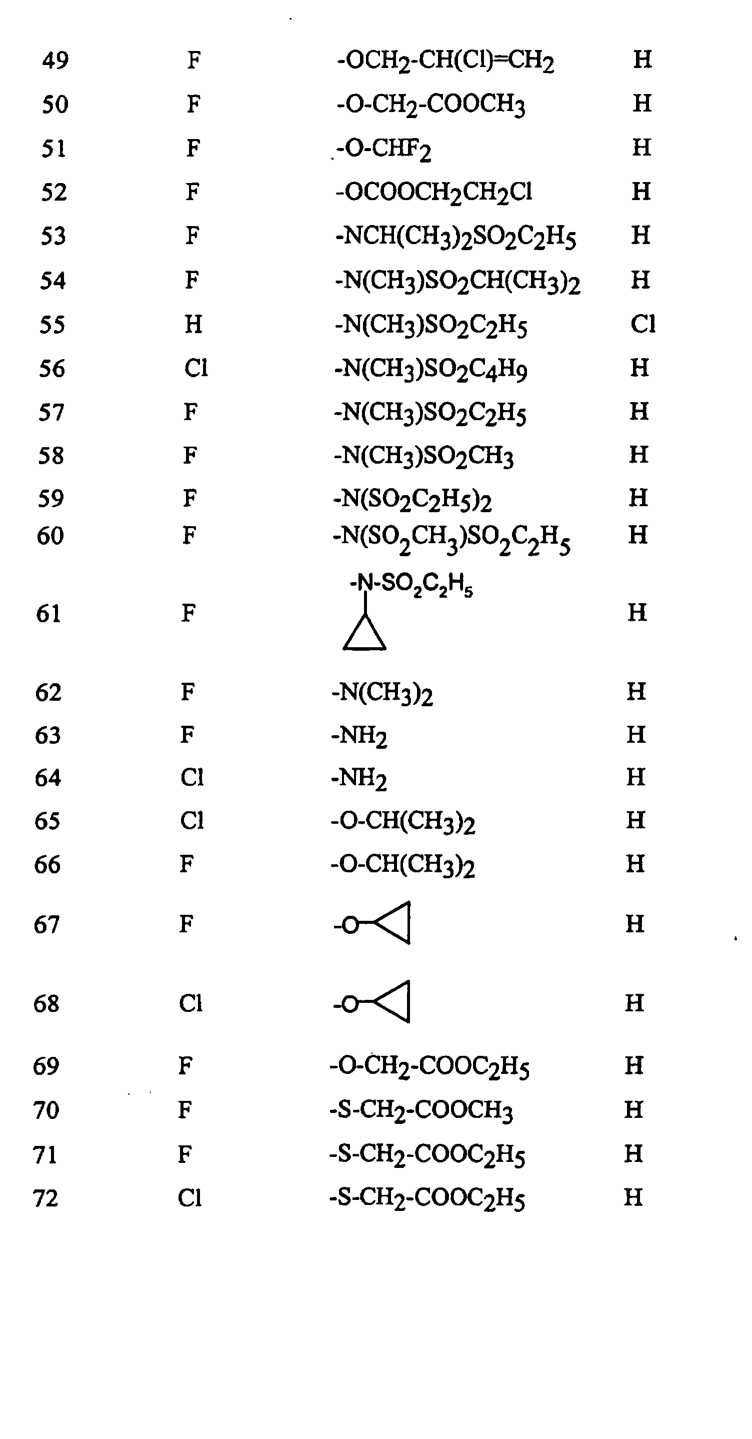

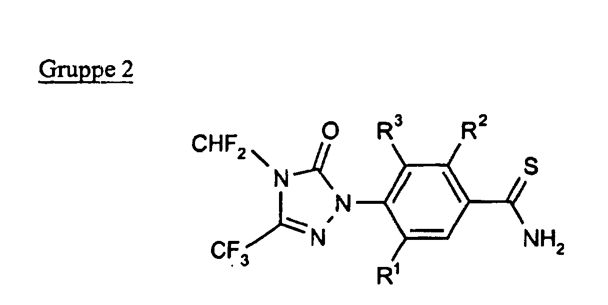

Verwendet man beispielsweise 2-(2-Fluor-4-cyano-5-methoxy-phenyl)-4-methyl-5-difluormethyl-2,4-dihydro-3H-1,2,4-triazol-3-on und Schwefelwasserstoff als Ausgangsstoffe, so kann der Reaktionsablauf des erfindungsgemäßen Verfahrens durch das folgende Formelschema skizziert werden:

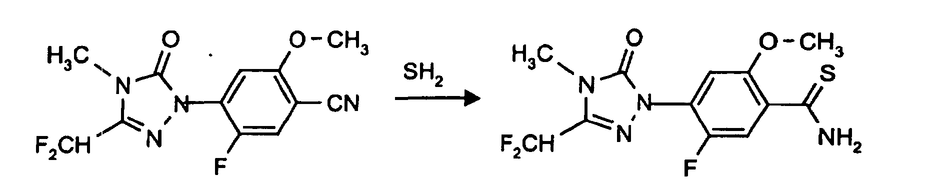

Die beim erfindungsgemäßen Verfahren zur Herstellung der Verbindungen der allgemeinen Formel (I) als Ausgangsstoffe zu verwendenden substituierten aromatischen Nitrile sind durch die Formel (II) allgemein definiert. In der Formel (II) haben R¹, R², R³ und Z vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹, R², R³ und Z angegeben wurden.

Die Ausgangsstoffe der Formel (II) sind bekannt und/oder können nach bekannten Verfahren hergestellt werden (vgl. EP-A 370332; DE-A 4238125; DE-A 4303376; US-P 5084084; Herstellungsbeispiele).

Als Verdünnungsmittel zur Durchführung des erfindungsgemäßen Verfahrens kommen die üblichen organischen Lösungsmittel in Betracht. Hierzu gehören insbesondere; aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäuremethylester oder Essigsäureethylester, Sulfoxide, wie Dimethylsulfoxid, Azine, wie Pyridin, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie basische organische Stickstoffverbindungen, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 0°C und 100°C, vorzugsweise bei Temperaturen zwischen 10°C und 80°C.

Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens werden die Ausgangsstoffe der Formel (II) im allgemeinen in einem geeigneten Verdünnungsmittel in Gegenwart eines Reaktionshilfsmittels vorgelegt und der Schwefelwasserstoff oder das Thioacetamid wird langsam eindosiert. Vorzugsweise werden der Schwefelwasserstoff oder das Thioacetamid in einem größeren Überschuss eingesetzt. Das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleurn, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf als auch im Nachauflauf-Verfahren.

In gewissem Umfang zeigen die Verbindungen der Formel (I) auch fungizide Wirkung, beispielsweise gegen Pyricularia oryzae an Reis.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche, oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop(-methyl), Fenoxaprop(-ethyl), Fluazifop(-butyl), Haloxyfop(-methyl) und Quizalofop(-ethyl); Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Chlormethoxynil (X-52), Chlomitrofen, Fluoroglycofen, Fomesafen, Halosafen, Lactofen, Nitrofen und Oxyfluorfen; Harnstoffe, wie z.B. Chlortoluron, Cumyluron (JC-940), Diuron, Dymron (Daimuron), Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. AC-014 (AC-322140), Amidosulfuron, Bensulfuron(-methyl), Chlorimuron(-ethyl), Chlorsulfuron, Cinosulfuron, DPX-47, HOE-404, Imazosulfuron, Metsulfuron(-methyl), Nicosulfuron, Primisulfuron, Pyrazosulfuron(-ethyl), Thifensulfuron(-methyl), Triasulfuron und Tribenuron(-methyl); Thiolcarbamate, wie z.B. Butylate, Cycloate, Diallate, Dimepiperate, EPTC, Esprocarb, Molinate, Prosulfocarb, Thiobencarb (Benthiocarb) und Triallate; Triazine, wie z.B. Atrazin, Cyanazin, Dimethametryn, Prometryne, Simazin, Simetryne, Terbutryne und Terbutylazin; Triazinone, wie z.B. Hexazinon, Metamitron und Metribuzin; Sonstige, wie z.B. Aminotriazol, Benfuresate, Bensulide, Bentazone, Benzofenap, Bromobutide, Butamifos, Cafenstrole (CH-900), Cinmethylin, Clomazone, Clomeprop, Clopyralid, DEH-112, Difenzoquat, Dimethenamid, Dithiopyr, Ethofumesate, Flumetsulam, Fluorochloridone, Glufosinate, Glyphosate, Amiprophos(-methyl), Anilofos, Etobenzanid (HW-52), Isoxaben, KPP-314, KUH-833, KUH-911, KUH-920, MK-243, Naproanilide, NSK-850, Oxadiazon, Piperophos, Propanil, Pyrazolate, Pyrazoxyfen, Pyributicarb, Pyridate, Quinchlorac, Quinmerac, Sulphosate und Tridiphane.

Auch eine Mischung mit anderen bekannten Wirkstoffen; wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

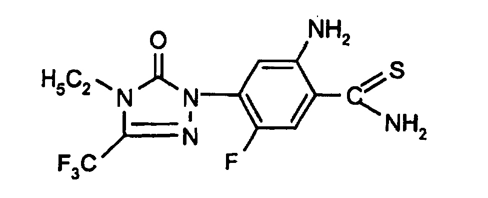

6,3 g (0,02 Mol) 2-(2-Fluor-4-cyano-5-amino-phenyl)-4-ethyl-5-trifluormethyl-2,4-dihydro-3H-1,2,4-triazol-3-on werden in 100 ml Aceton mit 4,04 g (0,04 Mol) Triethylamin versetzt. Bei 23°C wird nun zügig Schwefelwasserstoff eingeleitet, wobei die Innentemperatur bis auf 33°C ansteigt. Nach 1 Stunde ist die Reaktion vollständig. Die Lösung wird am Rotationsverdampfer eingeengt und der Rückstand aus Isopropanol umkristallisiert.

Man erhält 2,9 g (42% der Theorie) 2-(2-Fluor-4-thiocarbamoyl-5-amino-phenyl)-4-ethyl-5-trifluormethyl-2,4-dihydro-3H-1,2,4-thazol-3-on vom Schmelzpunkt 161°C.

### Beispiel 2

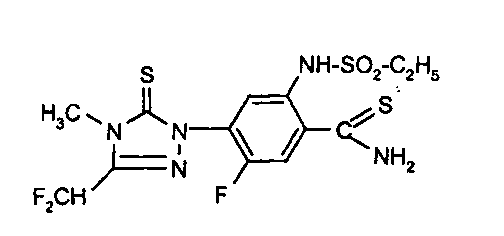

11 g (0,0276 Mol) 2-(2-Fluor-4-cyano-5-ethylsulfonylaminophenyl)-4-methyl-5-difluormethyl-2,4-dihydro-3H-1,2,4-triazol-3-thion werden in 100 ml Pyridin unter Einleiten von Schwefelwasserstoff 4,5 Stunden bei 70°C gerührt. Die Lösung wird am Rotationsverdampfer eingeengt, der Rückstand in Wasser verrührt, mit konzentrierter Salzsäure angesäuert, ausgefallenes Produkt abfiltriert, mit Wasser gewaschen und aus Isopropanol umkristallisiert.

Man erhält 9 g (77% der Theorie) 2-(2-Fluor-4-thiocarbamoyl-5-ethylsulfonylaminophenyl)-4-methyl-5-difluormethyl-2,4-dihydro-3H-1,2,4-triazol-3-thion vom Schmelzpunkt 183°C.

Analog zu den Herstellungsbeispielen 1 und 2 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

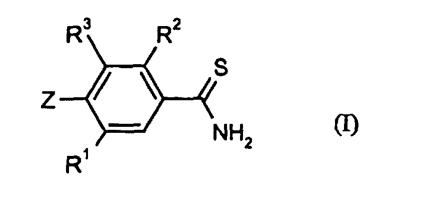

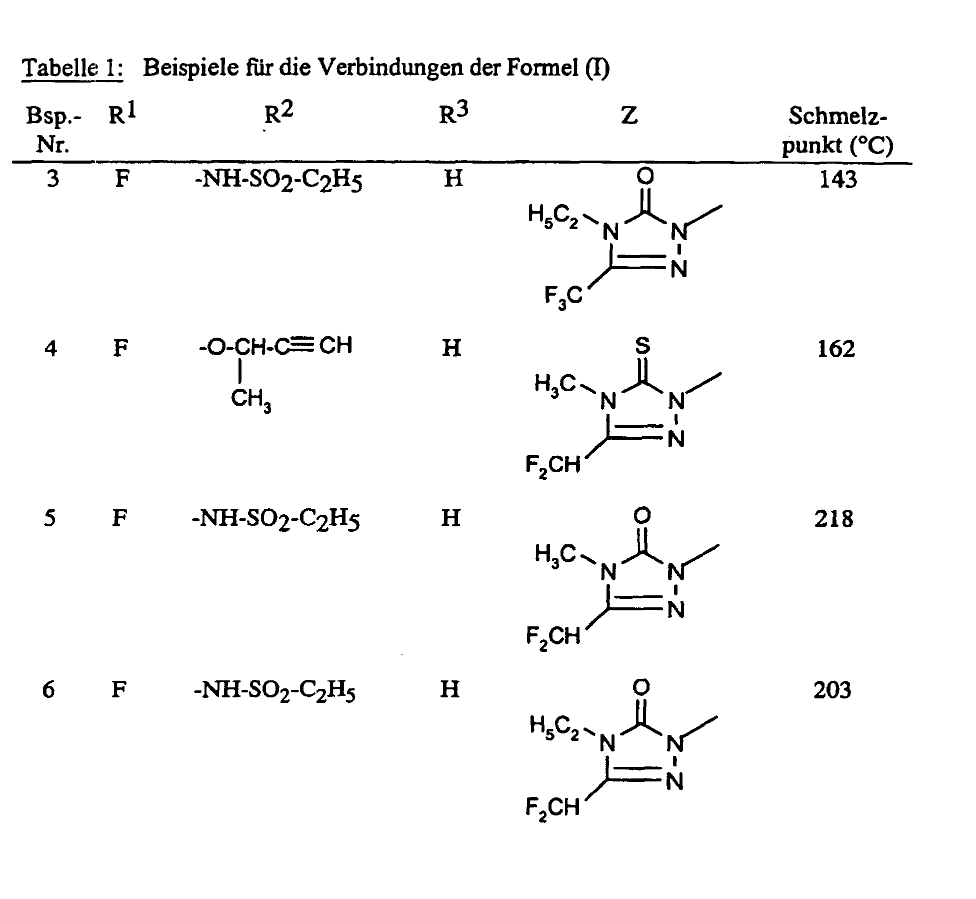

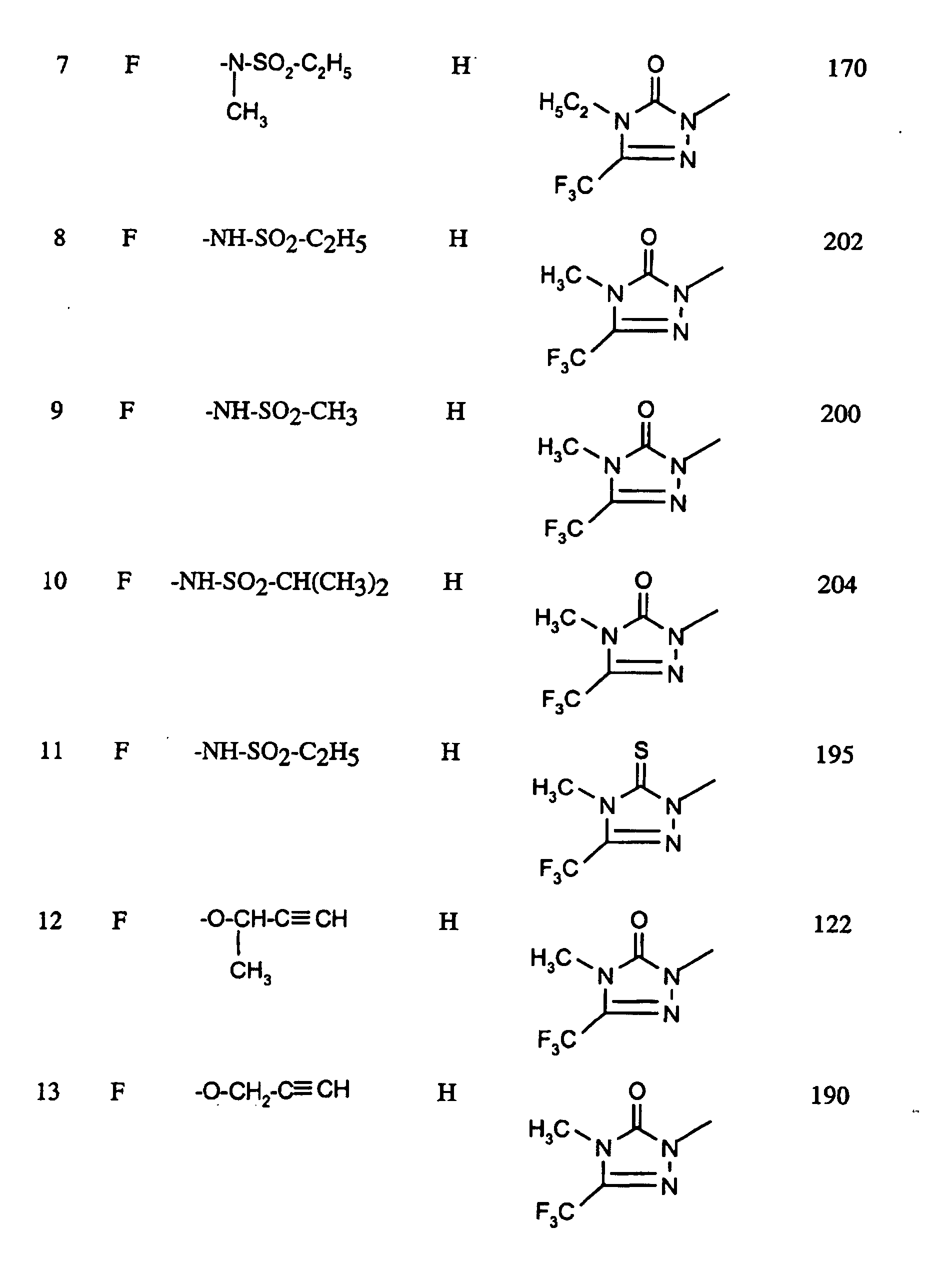

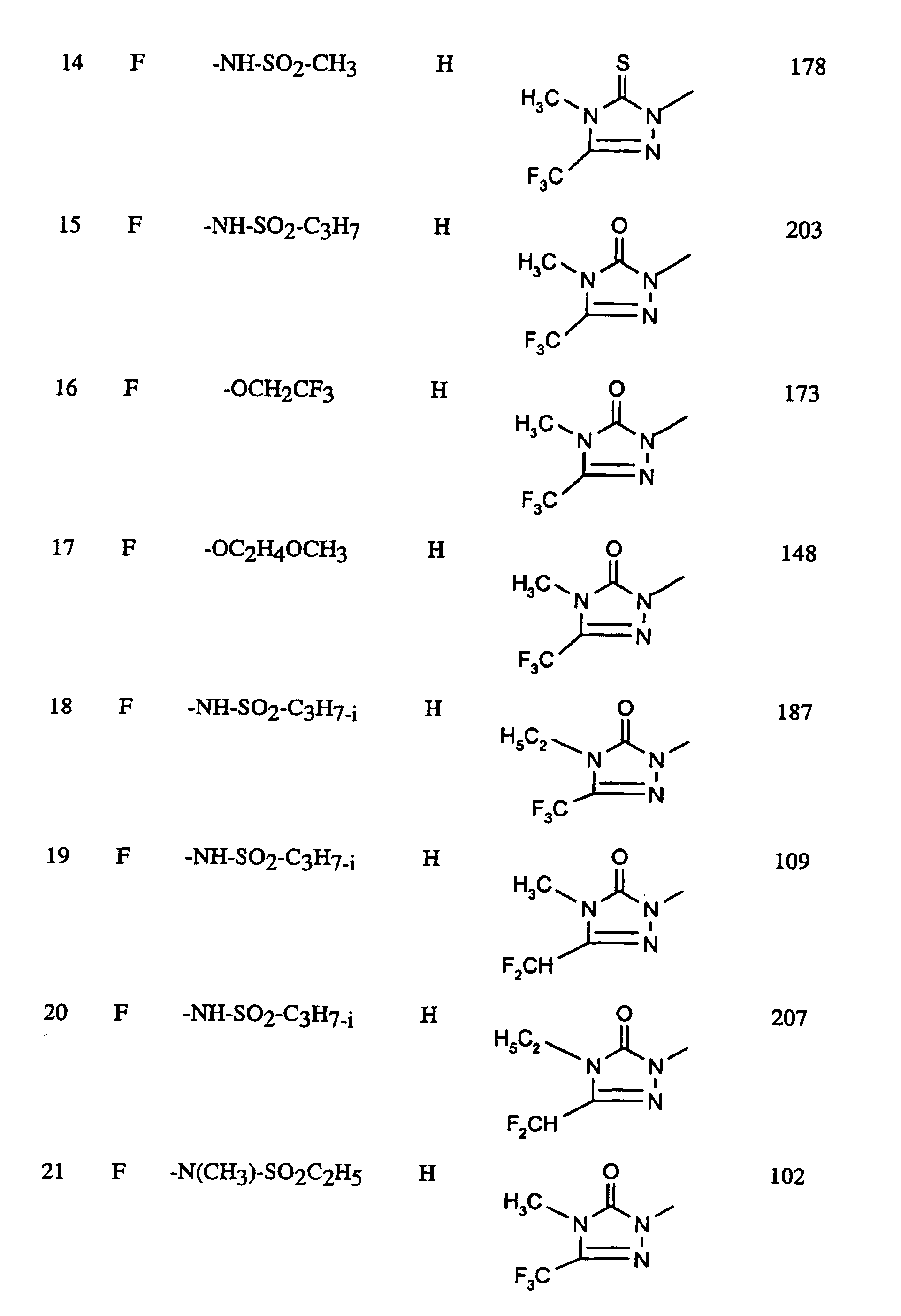

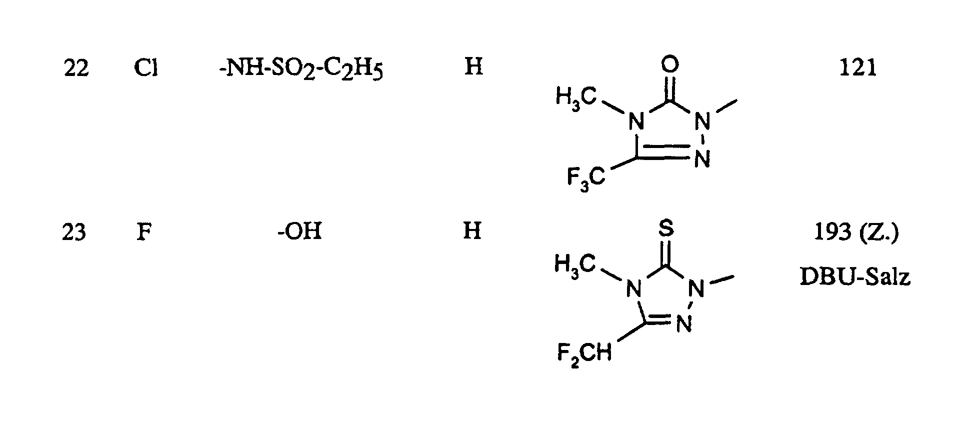

### Herstellung der Ausgangsverbindungen:

### Beispiel II-1:

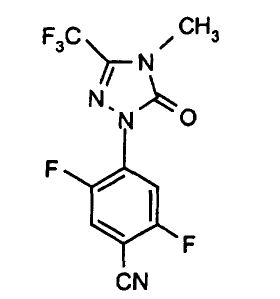

Zu 6,3 g (0,034 Mol) 4-Methyl-3-trifluormethyl-1,2,4-triazolin-5-on (vgl. z.B. US 3.780.052) und 5,4 g (0,034 Mol) 2,4,5-Trifluorbenzonitril (vgl. z.B. EP 191181) in 150 ml Dimethylsulfoxid gibt man bei Raumtemperatur 5,8 g (0,042 Mol) Kaliumcarbonat und erwärmt anschließend für 14 Stunden auf 100°C. Zur Aufarbeitung wird die abgekühlte Reaktionsmischung in Wasser gegeben, mit verdünnter Salzsäure auf pH 2 gebracht und mehrfach mit Dichlormethan extrahiert. Die vereinigten organischen Phasen werden über Natriumsulfat getrocknet und im Vakuum eingeengt. Der Rückstand wird über Kieselgel (Laufmittel: Dichlormethan) chromatographiert.

Man erhält 6,2 g (60% der Theorie) 1-(4-Cyano-2,5-difluorphenyl)-4-methyl-3-trifluormethyl-1,2,4-triazolin-5-on vom Schmelzpunkt 74°C.

### Beispiel II-2

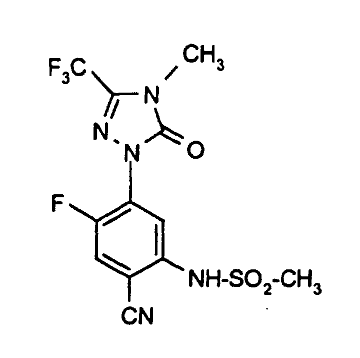

Zu 1,52 g (0,005 Mol) 1-(4-Cyano-2,5-difluorphenyl)-4-methyl-3-trifluormethyl-1,2,4-triazolin-5-on und 0,48 g (0,005 Mol) Methansulfonsäureamid in 50 ml Dimethylsulfoxid gibt man bei Raumtemperatur 0,83 g (0,006 Mol) Kaliumcarbonat und erwärmt anschließend für 12 Stunden auf 120°C. Zur Aufarbeitung wird die abgekühlte Reaktionsmischung in Wasser gegeben, mit verdünnter Salzsäure auf pH 2 gebracht und mehrfach mit Dichlormethan extrahiert. Die vereinigten organischen Phasen werden über Natriumsulfat getrocknet und im Vakuum eingeengt. Der Rückstand wird über Kieselgel (Laufmittel: Dichlormethan/Methanol 20: 1) chromatographiert.

Man erhält 0,55 g (28% der Theorie) 1-(4-Cyano-2-fluor-5-methylsulfonylaminophenyl)-4-methyl-3-trifluormethyl-1,2,4-tnazolin-5-on vom Schmelzpunkt 67°C.

**Tabelle A**

| Pre-emergence-Test/Gewächshaus | | | | | | | | |
|---|---|---|---|---|---|---|---|---|
| Wirkstoff (Synthese-beispiel Nummer) | Aufwand -menge (g/ha) | Gerste | Mais | Amaranthus | Chenopodium | Matricaria | Portulaca | Solanum |
| (2) | 125 | 0 | 0 | 100 | 100 | 100 | 100 | 100 |
| (3) | 125 | 0 | 0 | 100 | 100 | 90 | 90 | 100 |
| (4) | 125 | 0 | 30 | 100 | 100 | 95 | 100 | 100 |

**Tabelle B**

| Pre-emergence-Test/Gewächshaus | | | | | | | | | |
|---|---|---|---|---|---|---|---|---|---|
| Wirkstoff (Synthese -beispiel Nummer) | Aufwandmenge (g/ha) | Weizen | Mais | Abuthilon | Amaranthus | Chenopodium | Matricaria | Portulaca | Solanum |
| 3 | 125 | 0 | 0 | 10 | 100 | 100 | 70 | 90 | 100 |
| 2 | 60 | 0 | 0 | 100 | 100 | 100 | 100 | 100 | 100 |
| 4 | 60 | 20 | 30 | 100 | 100 | 100 | 100 | 100 | 95 |
| 5 | 60 | 0 | 0 | 100 | 100 | 100 | 95 | 90 | 100 |
| 7 | 60 | 10 | 0 | 95 | 20 | 100 | 90 | 80 | 80 |
| 8 | 60 | 0 | 20 | 100 | 100 | 100 | 100 | 100 | 100 |
| 9 | 60 | 0 | 0 | 100 | 100 | 100 | 100 | 100 | 100 |
| 10 | 60 | 0 | 0 | 100 | 100 | 100 | 90 | 100 | 100 |
| 11 | 60 | 0 | 0 | 100 | 100 | 100 | 100 | 100 | 100 |
| 12 | 30 | 10 | 0 | - | 100 | 100 | 100 | 95 | 100 |
| 13 | 30 | 10 | 10 | 95 | 95 | 100 | 100 | 100 | 100 |
| 14 | 60 | 0 | 0 | 100 | 100 | 100 | 100 | 100 | 100 |
| 15 | 60 | 0 | 0 | 100 | 100 | 100 | 90 | 95 | 100 |
| 16 | 60 | 20 | 30 | 100 | 100 | 100 | 100 | 100 | 100 |

### Beispiel B

Post-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben, so dass die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.
Es bedeuten:
- 0 % =: keine Wirkung (wie unbehandelte Kontrolle)
- 100 % =: totale Vernichtung

**Tabelle C**

| Post-emergence-Test/Gewächshaus | | | | | | | | |
|---|---|---|---|---|---|---|---|---|
| Wirkstoff (Synthese -beispiel Nummer) | Aufwandmenge (g/ha) | Weizen | Mais | Abuthilon | Amaranthus | Chenopodium | Solanum | Veronica |
| 3 | 15 | 10 | 20 | 100 | 100 | 100 | 100 | 100 |
| 2 | 30 | 10 | 30 | 100 | 100 | 100 | 100 | 100 |
| 4 | 8 | 30 | 50 | 100 | 100 | 100 | 100 | 95 |
| 5 | 60 | 10 | 30 | 100 | 100 | 100 | 100 | 100 |
| 7 | 60 | 20 | 60 | 95 | 100 | 100 | 100 | 100 |
| 8 | 30 | 0 | 30 | 100 | 100 | 90 | 100 | 100 |
| 9 | 8 | 5 | 0 | 70 | 100 | 90 | 100 | 95 |
| 10 | 8 | 0 | 50 | 100 | 100 | 95 | 100 | 70 |
| 11 | 8 | 0 | 70 | 100 | 100 | 90 | 100 | 100 |
| 12 | 4 | 15 | 30 | 100 | 100 | 95 | 100 | 100 |
| 13 | 4 | 20 | 60 | 100 | 100 | 100 | 100 | 100 |
| 14 | 15 | 0 | 30 | 100 | 100 | 100 | 100 | 100 |
| 15 | 30 | 0 | 25 | 100 | 100 | 90 | 100 | 95 |
| 16 | 15 | 20 | 50 | 100 | 80 | 90 | 100 | 95 |

## Patentansprüche

1. Substituierte aromatische Thiocarbonsäureamide der allgemeinen Formel (I), 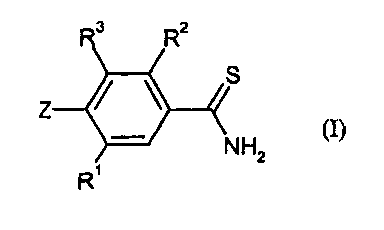 in welcher
R¹ für Wasserstoff, Fluor, Chlor oder Brom steht,
R² für die nachstehende Gruppierung steht,
-A¹-A³
in welcher
A¹ für Sauerstoff, Schwefel oder die Gruppierung -N-A⁴- steht, worin A⁴ für Wasserstoff, C₁-C₄-Alkyl, C₁-C₄-Alkoxy oder C₁-C₄-Alkylsulfonyl steht,
A³ für Wasserstoff, Hydroxy, Amino, Cyano, Isocyanato, Thiocyanato, Nitro, Carboxy, Carbamoyl, Thiocarbamoyl, Sulfo, Chlorsulfonyl, Halogen, für jeweils gegebenenfalls durch Halogen oder C₁-C₄-Alkoxy substituiertes Alkyl, Alkoxy, Alkylthio, Alkylsulfinyl, Alkylsulfonyl, Alkylamino, Dialkylamino, Alkoxycarbonyl oder Dialkoxy(thio)phosphoryl mit jeweils 1 bis 6 Kohlenstoffatomen in den Alkylgruppen, für jeweils gegebenenfalls durch Halogen substituiertes Alkenyl, Alkenyloxy, Alkenylamino, Alkylidenamino, Alkenyloxycarbonyl, Alkinyl, Alkinyloxy, Alkinylamino oder Alkinyloxycarbonyl mit jeweils 2 bis 6 Kohlenstoffatomen in den Alkenyl-, Alkyliden- oder Alkinylgruppen, für jeweils gegebenenfalls durch Halogen, Cyano, Carboxy, C₁-C₄-Alkyl und/oder C₁-C₄-Alkoxycarbonyl substituiertes Cycloalkyl, Cycloalkyloxy, Cycloalkylalkyl, Cycloalkylalkoxy, Cycloalkylidenamino, Cycloalkyloxycarbonyl oder Cycloalkylalkoxycarbonyl mit jeweils 3 bis 6 Kohlenstoffatomen in den Cycloalkylgruppen und gegebenenfalls 1 bis 4 Kohlenstoffatomen in den Alkylgruppen, oder für jeweils gegebenenfalls durch Nitro, Cyano, Carboxy, Halogen, C₁-C₄-Alkyl, C₁-C₄-Halogenalkyl, C₁-C₄-Alkyloxy, C₁-C₄-Halogenalkyloxy und/oder C₁-C₄-Alkoxycarbonyl substituiertes Phenyl, Phenyloxy, Phenyl-C₁-C₄-alkyl, Phenyl-C₁-C₄-alkoxy, Phenyloxycarbonyl oder Phenyl-C₁-C₄-alkoxycarbonyl, (jeweils gegebenenfalls ganz oder teilweise hydriertes) Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolyl-C₁-C₄-alkyl, Furyl-C₁-C₄-alkyl, Thienyl-C₁-C₄-alkyl, Oxazolyl-C₁-C₄-alkyl, Isoxazol-C₁-C₄-alkyl, Thiazol-C₁-C₄-alkyl, Pyridinyl-C₁-C₄-alkyl, Isoxazol-C₁-C₄-alkyl, Thiazol-C₁-C₄-alkyl, Pyridinyl-C₁-C₄-alkyl, Pyrimidinyl-C₁-C₄-alkyl, Pyrazolylmethoxy, Furylmethoxy, für Perhydropyranylmethoxy oder Pyridylmethoxy steht,
R³ für Wasserstoff, Fluor, Chlor oder Brom steht, und
Z für die nachstehend aufgeführte heterocyclische Gruppierung steht, 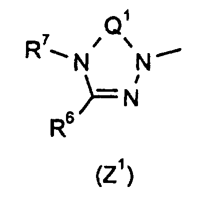
wobei
Q¹ für -CO- oder -CS- steht,
R⁶ für Difluormethyl oder Trifluormethyl steht, und
R⁷ für Methyl, Ethyl, n- oder i-Propyl oder Difluormethyl steht.

2. Substituierte aromatische Thiocarbonsäureamide gemäß Anspruch 1, **dadurch gekennzeichnet, dass**
R¹ für Wasserstoff, Fluor oder Chlor steht,
R² für die nachstehende Gruppierung steht,
-A¹-A³
in welcher
A¹ für Sauerstoff, Schwefel oder die Gruppierung -N-A⁴- steht, worin A⁴ für Wasserstoff, Methyl, Ethyl, n- oder i-Propyl, Methoxy, Ethoxy, n- oder i-Propoxy, Methylsulfonyl oder Ethylsulfonyl steht,
A³ für Wasserstoff, Hydroxy, Amino, Cyano, Nitro, Carboxy, Carbamoyl, Sulfo, Fluor, Chlor, Brom, für jeweils gegebenenfalls durch Fluor, Chlor, Methoxy oder Ethoxy substituiertes Methyl, Ethyl, n- oder i-Propyl, n-, i-, s- oder t-Butyl, n-, i-, s- oder t-Pentyl, Methoxy, Ethoxy, n- oder i-Propoxy, n-, i-, s- oder t-Butoxy, n-, i-, s-oder t-Pentyloxy, Methylthio, Ethylthio, n- oder i-Propylthio, n-, i-, s-oder t-Butylthio, Methylsulfinyl, Ethylsulfinyl, n- oder i-Propyl-sulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Methoxycarbonyl, Ethoxycarbonyl, n- oder i-Propoxycarbonyl, Dimethoxyphosphoryl, Diethoxyphosphoryl, Dipropoxyphosphoryl oder Diisopropoxyphosphoryl, für jeweils gegebenenfalls durch Fluor oder Chlor substituiertes Propenyl, Butenyl, Propenyloxy, Butenyloxy, Propenylamino, Butenylamino, Propylidenamino, Butylidenamino, Propenyloxycarbonyl, Butenyloxycarbonyl, Propinyl, Butinyl, Propinyloxy, Butinyloxy, Propinylamino, Butinylamino, Propinyloxycarbonyl oder Butinyloxycarbonyl, für jeweils gegebenenfalls durch Fluor, Chlor, Cyano, Carboxy, Methyl, Ethyl, n- oder i-Propyl, Methoxycarbonyl oder Ethoxycarbonyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopentylidenamino, Cyclohexylidenamino, Cyclopentyloxycarbonyl, Cyclohexyloxycarbonyl, Cyclopentylmethoxycarbonyl oder Cyclohexylmethoxycarbonyl, oder für jeweils gegebenenfalls durch Nitro, Cyano, Carboxy, Fluor, Chlor, Brom, Methyl, Ethyl, n- oder i-Propyl, Trifluormethyl, Methoxy, Ethoxy, n- oder i-Propoxy, Difluormethoxy, Trifluormethoxy, Methoxycarbonyl und/oder Ethoxycarbonyl substituiertes Phenyl, Phenyloxy, Benzyl, Phenylethyl, Benzyloxy, Phenyloxycarbonyl, Benzyloxycarbonyl, (jeweils gegebenenfalls ganz oder teilweise hydriertes) Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolylmethyl, Furylmethyl, Thienylmethyl, Oxazolylmethyl, Isoxazolmethyl, Thiazolmethyl, Pyridinylmethyl, Pyrimidinylmethyl, Pyrazolylmethoxy, Furylmethoxy oder Pyridylmethoxy steht,
R³ für Wasserstoff, Fluor oder Chlor steht.

3. Verbindung gemäß Anspruch 1, **dadurch gekennzeichnet, dass**
R¹ für Fluor steht,
R² für -NH-SO₂-C₂H₅ steht,
R³ für Wasserstoff steht,
Q¹ für -CO- steht,
R⁶ für Trifluormethyl steht, und
R⁷ für Methyl steht.

4. Verfahren zur Herstellung von substituierten aromatischen Thiocarbonsäureamiden, gemäß einem der Ansprüche 1 bis 3, **dadurch gekennzeichnet, dass** man substituierte aromatische Nitrile der allgemeinen Formel (II) 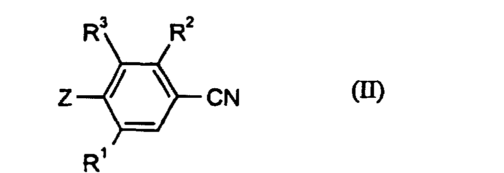 in welcher
R¹, R², R³ und Z die in einem der Ansprüche 1 bis 3 angegebenen Bedeutungen haben,
mit Schwefelwasserstoff (Hydrogensulfid, H₂S) oder mit Thioacetamid gegebenenfalls in Gegenwart eines Reaktionshilfsmittels und gegebenenfalls in Gegenwart eines Verdünnungsmittels umsetzt.

5. Verfahren zur Bekämpfung von unerwünschten Pflanzen, **dadurch gekennzeichnet, dass** man substituierte aromatische Thiocarbonsäureamide gemäß einem der Ansprüche 1 bis 3 auf unerwünschte Pflanzen und/oder ihren Lebensraum einwirken lässt.

6. Verwendung von substituierten aromatischen Thiocarbonsäureamiden gemäß einem der Ansprüche 1 bis 3 zur Bekämpfung von unerwünschten Pflanzen.

7. Verfahren zur Herstellung von herbiziden Mitteln, **dadurch gekennzeichnet, dass** man substituierte aromatische Thiocarbonsäureamide gemäß einem der Ansprüche 1 bis 3 mit Streckmitteln und/oder oberflächenaktiven Substanzen vermischt.

8. Herbizide Mittel, **gekennzeichnet durch** einen Gehalt an mindestens einem substituierten aromatischen Thiocarbonsäureamid gemäß einem der Ansprüche 1 bis 3.

## Claims

1. Substituted aromatic thiocarboxamides of the general formula (I) 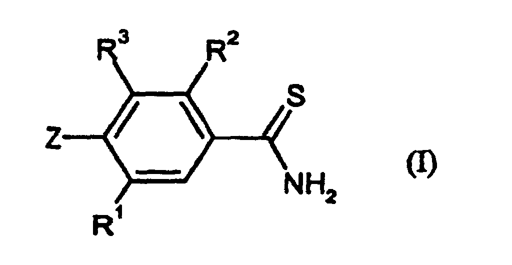 in which
R¹ represents hydrogen, fluorine, chlorine or bromine,
R² represents the following group
-A¹-A³
in which
A¹ represents oxygen, sulphur, or the group -N-A⁴-, in which A⁴ represents hydrogen, C₁-C₄-alkyl, C₁-C₄-alkoxy or C₁-C₄-alkylsulphonyl,
A³ represents hydrogen, hydroxyl, amino, cyano, isocyano, thiocyanato, nitro, carboxyl, carbamoyl, thiocarbamoyl, sulpho, chlorosulphonyl, halogen, or represents in each case optionally halogen- or C₁-C₄-alkoxy-substituted alkyl, alkoxy, alkylthio, alkylsulphinyl, alkylsulphonyl, alkylamino, dialkylamino, alkoxycarbonyl or dialkoxy(thio)phosphoryl having in each case 1 to 6 carbon atoms in the alkyl groups, or represents in each case optionally halogen-substituted alkenyl, alkenyloxy, alkenylamino, alkylideneamino, alkenyloxycarbonyl, alkinyl, alkinyloxy, alkinylamino or alkinyloxycarbonyl having in each case 2 to 6 carbon atoms in the alkenyl, alkylidene or alkinyl groups, or represents in each case optionally halogen-, cyano-, carboxyl-, C₁-C₄-alkyl- and/or C₁-C₄-alkoxy-carbonyl-substituted cycloalkyl, cycloalkyloxy, cycloalkylalkyl, cycloalkylalkoxy, cycloalkylideneamino, cycloalkyloxycarbonyl or cycloalkylalkoxycarbonyl having in each case 3 to 6 carbon atoms in the cycloalkyl groups and optionally 1 to 4 carbon atoms in the alkyl groups, or represents in each case optionally nitro-, cyano-, carboxyl-, halogen-, C₁-C₄-alkyl-, C₁-C₄-halogenoalkyl-, C₁-C₄-alkyloxy-, C₁-C₄-halogenoalkyloxy- and/or C₁-C₄-alkoxy-carbonyl-substituted phenyl, phenyloxy, phenyl-C₁-C₄-alkyl, phenyl-C₁-C₄-alkoxy, phenyloxycarbonyl or phenyl-C₁-C₄-alkoxycarbonyl, (in each case optionally totally or partially hydrogenated) pyrrolyl, pyrazolyl, imidazolyl, triazolyl, furyl, thienyl, oxazolyl, isoxazolyl, thiazolyl, isothiazolyl, oxadiazolyl, thiadiazolyl, pyridinyl, pyrimidinyl, triazinyl, pyrazolyl-C₁-C₄-alkyl, furyl-C₁-C₄-alkyl, thienyl-C₁-C₄-alkyl, oxazolyl-C₁-C₄-alkyl, isoxazole-C₁-C₄-alkyl, thiazole-C₁-C₄-alkyl, pyridinyl-C₁-C₄-alkyl, pyrimidinyl-C₁-C₄-alkyl, pyrazolylmethoxy or furylmethoxy, or represents perhydropyranylmethoxy or pyridylmethoxy,
R³ represents hydrogen, fluorine, chlorine or bromine and
Z represents the heterocyclic moiety set out below, 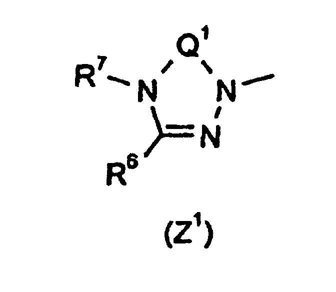
where
Q¹ represents -CO- or -CS-,
R⁶ represents difluoromethyl or trifluoromethyl, and
R⁷ represents methyl, ethyl, n- or i-propyl or difluoromethyl.

2. Substituted aromatic thiocarboxamides according to Claim 1, **characterized in that**
R¹ represents hydrogen, fluorine or chlorine,
R² represents the following group
-A¹-A³
in which
A¹ represents oxygen, sulphur, or the group -N-A⁴-, in which A⁴ represents hydrogen, methyl, ethyl, n- or i-propyl, methoxy, ethoxy, n- or i-propoxy, methylsulphonyl or ethylsulphonyl,
A³ represents hydrogen, hydroxyl, amino, cyano, nitro, carboxyl, carbamoyl, sulpho, fluorine, chlorine, bromine, or represents in each case optionally fluorine-, chlorine-, methoxy- or ethoxy-substituted methyl, ethyl, n- or i-propyl, n-, i-, s- or t-butyl, n-, i-, s- or t-pentyl, methoxy, ethoxy, n- or i-propoxy, n-, i-, s- or t-butoxy, n-, i-, s- or t-pentyloxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, methylamino, ethylamino, n- or i-propylamino, n-, i-, s- or t-butylamino, dimethylamino, diethylamino, methoxycarbonyl, ethoxycarbonyl, n- or i-propoxycarbonyl, dimethoxyphosphoryl, diethoxyphosphoryl, dipropoxyphosphoryl or diisopropoxyphosphoryl, or represents in each case optionally fluorine-or chlorine-substituted propenyl, butenyl, propenyloxy, butenyloxy, propenylamino, butenylamino, propylideneamino, butylideneamino, propenyloxycarbonyl, butenyloxycarbonyl, propinyl, butinyl, propinyloxy, butinyloxy, propinylamino, butinylamino, propinyloxycarbonyl or butinyloxy-carbonyl, or represents in each case optionally fluorine-, chlorine-, cyano-, carboxyl-, methyl-, ethyl-, n- or i-propyl-, methoxycarbonyl- or ethoxycarbonyl-substituted cyclopropyl, cyclobutyl, cyclopentyl, cyclohexyl, cyclopropyloxy, cyclobutyloxy, cyclopentyloxy, cyclohexyloxy, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopentylideneamino, cyclohexylideneamino, cyclopentyloxycarbonyl, cyclohexyloxycarbonyl, cyclopentylmethoxycarbonyl or cyclohexylmethoxycarbonyl, or represents in each case optionally nitro-, cyano-, carboxyl-, fluorine-, chlorine-, bromine-, methyl-, ethyl-, n- or i-propyl-, trifluoromethyl-, methoxy-, ethoxy-, n- or i-propoxy-, difluoromethoxy-, trifluoromethoxy-, methoxy-carbonyl- and/or ethoxycarbonyl-substituted phenyl, phenyloxy, benzyl, phenylethyl, benzyloxy, phenyloxycarbonyl, benzyloxycarbonyl, (in each case optionally completely or partially hydrogenated) pyrrolyl, pyrazolyl, imidazolyl, triazolyl, furyl, thienyl, oxazolyl, isoxazolyl, thiazolyl, isothiazolyl, oxadiazolyl, thiadiazolyl, pyridinyl, pyrimidinyl, triazinyl, pyrazolylmethyl, furylmethyl, thienylmethyl, oxazolylmethyl, isoxazolemethyl, thiazolmethyl, pyridinylmethyl, pyrimidinylmethyl, pyrazolylmethoxy, furylmethoxy or pyridylmethoxy,
R³ represents hydrogen, fluorine or chlorine.

3. Compound according to Claim 1, **characterized in that**
R¹ represents fluorine,
R² represents -NH-SO₂-C₂H₅,
R³ represents hydrogen,
Q¹ represents -CO-,
R⁶ represents trifluoromethyl, and
R⁷ represents methyl.

4. Process for the preparation of substituted aromatic thiocarboxamides according to one of Claims 1 to 3, **characterized in that** substituted aromatic nitriles of the general formula (II) 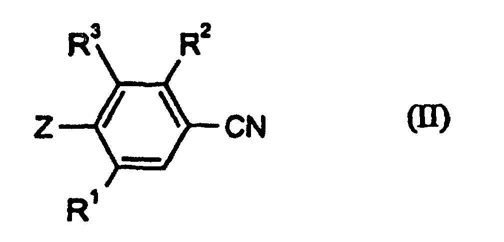 in which
R¹, R², R³ and Z have the meanings indicated in any of Claims 1 to 3
are reacted with hydrogen sulphide (H₂S) or with thioacetamide, optionally in the presence of a reaction auxiliary and optionally in the presence of a diluent.

5. Method of combatting unwanted plants, **characterized in that** substituted aromatic thiocarboxamides according to any of Claims 1 to 3 are caused to act on unwanted plants and/or their habitat.

6. Use of substituted aromatic thiocarboxamides according to any of Claims 1 to 3 for combatting unwanted plants.

7. Process for the production of herbicidal compositions, **characterized in that** substituted aromatic thiocarboxamides according to any of Claims 1 to 3 are mixed with extenders and/or surfactants.

8. Herbicidal compositions, **characterized by** a content of at least one substituted aromatic thiocarboxamide according to any of Claims 1 to 3.

## Revendications

1. Amides d'acides thiocarboxyliques aromatiques substitués de formule générale (I) 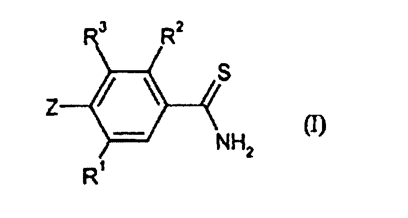 dans laquelle
R¹ représente l'hydrogène, le fluor, le chlore ou le brome,
R² représente le groupement suivant,
-A¹-A³
dans lequel
A¹ représente l'oxygène, le soufre ou le groupement -N-A⁴-, où A⁴ représente l'hydrogène, un reste alkyle en C₁ à C₄, alkoxy en C₁ à C₄ ou alkylsulfonyle en C₁ à C₄,
A³ représente l'hydrogène, un groupe hydroxy, amino, cyano, isocyanato, thiocyanato, nitro, carboxy, carbamoyle, thiocarbamoyle, sulfo, chlorosulfonyle, un halogène, un reste alkyle, alkoxy, alkylthio, alkylsulfinyle, alkylsulfonyle, alkylamino, dialkylamino, alkoxycarbonyle ou dialkoxy(thio)phosphoryle ayant chacun 1 à 6 atomes de carbone dans les groupes alkyle, et chacun étant éventuellement substitué par un radical halogéno ou alkoxy en C₁ à C₄, un reste alcényle, alcényloxy, alcénylamino, alkylidèneamino, alcényloxycarbonyle, alcynyle, alcynyloxy, alcynylamino ou alcynyloxycarbonyle ayant chacun 2 à 6 atomes de carbone dans les groupes alcényle, alkylidène ou alcynyle, et chacun étant éventuellement substitué par un halogène, un reste cycloalkyle, cycloalkyloxy, cycloalkylalkyle, cycloalkylalkoxy, cycloalkylidèneamino, cycloalkyloxycarbonyle ou cycloalkylalkoxycarbonyle ayant chacun 3 à 6 atomes de carbone dans les groupes cycloalkyle et, le cas échéant, 1 à 4 atomes de carbone dans les groupes alkyle et chacun étant éventuellement substitué par un radical halogène, cyano, carboxy, alkyle en C₁ à C₄ et/ou (alkoxy en C₁ à C₄)carbonyle, ou un reste phényle, phényloxy, phényl-(alkyle en C₁ à C₄), phényl-(alkoxy en C₁ à C₄), phényloxycarbonyle ou phényl-(alkoxy en C₁ à C₄)carbonyle, chacun éventuellement substitué par un radical nitro, cyano, carboxy, halogèno, alkyle en C₁ à C₄, halogénalkyle en C₁ à C₄, alkyloxy en C₁ à C₄, halogénalkyloxy en C₁ à C₄ et/ou (alkoxy en C₁ à C₄)-carbonyle, un reste (éventuellement hydrogéné en totalité ou en partie dans chaque cas) pyrrolyle, pyrazolyle, imidazolyle, triazolyle, furyle, thiényle, oxazolyle, isoxazolyle, thiazolyle, isothiazolyle, oxadiazolyle, thiadiazolyle, pyridinyle, pyrimidinyle, triazinyle, pyrazolyl-(alkyle en C₁ à C₄), furyl-(alkyle en C₁ à C₄), thiényl- (alkyle en C₁ à C₄), oxazolyl-(alkyle en C₁ à C₄), isoxazole-(alkyle en C₁ à C₄), thiazole-(alkyle en C₁ à C₄), pyridinyl- (alkyle en C₁ à C₄), isoxazole- (alkyle en C₁ à C₄), thiazole-(alkyle en C₁ à C₄), pyridinyl-(alkyle en C₁ à C₄), pyrimidinyl-(alkyle en C₁ à C₄), pyrazolylméthoxy, furylméthoxy, un reste perhydropyrannylméthoxy ou pyridylméthoxy,
R³ représente l'hydrogène, le fluor, le chlore ou le brome, et
Z représente le groupement hétérocyclique indiqué ci-après, 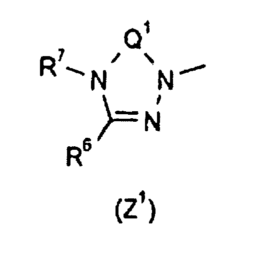
dans lequel
Q¹ est un groupe -CO- ou -CS-,
R⁶ est un reste difluorométhyle ou trifluorométhyle, et
R⁷ est un reste méthyle, éthyle, n-propyle, isopropyle ou difluorométhyle.

2. Amides d'acides thiocarboxyliques aromatiques substitués suivant la revendication 1, **caractérisés en ce que**
R¹ représente l'hydrogène, le fluor ou le chlore,
R² représente le groupement suivant,
-A¹-A³
dans lequel
A¹ représente l'oxygène, le soufre ou le groupement -N-A⁴-, où A⁴ est l'hydrogène, un reste méthyle, éthyle, n-propyle, isopropyle, méthoxy, éthoxy, n-propoxy, isopropoxy, méthylsulfonyle ou éthyl-sulfonyle,
A³ représente l'hydrogène, un groupe hydroxy, amino, cyano, nitro, carboxy, carbamoyle, sulfo, le fluor, le chlore, le brome, un reste méthyle, éthyle, n-propyle, isopropyle, n-butyle, isobutyle, sec.-butyle, tertiobutyle, n-pentyle, isopentyle, sec.-pentyle, tertiopentyle, méthoxy, éthoxy, n-propoxy, isopropoxy, n-butoxy, isobutoxy, sec.-butoxy, tertio-butoxy, n-pentyloxy, isopentyloxy, sec.-pentyloxy, tertiopentyloxy, méthylthio, éthylthio, n-propylthio, iso-propylthio, n-butylthio, isobutylthio, sec.-butylthio, propylthio, n-butylthio, isobutylthio, sec.-butylthio, tertio-butylthio, méthylsulfinyle, éthylsulfinyle, n-propylsulfinyle, isopropylsulfinyle, méthylsulfonyle, éthylsulfonyle, n-propylsulfonyle, isopropylsulfonyle, méthylamino, éthylamino, n-propylamino, isopropyl-amino, n-butylamino, isobutylamino, sec.-butylamino, tertio-butylamino, diméthylamino, diéthylamino, méthoxycarbonyle, éthoxycarbonyle, n-propoxycarbonyle, isopropoxycarbonyle, diméthoxyphosphoryle, diéthoxy-phosphoryle, dipropoxyphosphoryle ou diisopropoxy-phosphoryle chacun éventuellement substitué par un radical fluoro, chloro, méthoxy ou éthoxy, un reste propényle, butényle, propényloxy, butényloxy, propénylamino, buténylamino, propylidène-amino, butylidène-amino, propényloxycarbonyle, butényloxy-carbonyle, propynyle, butynyle, propynyloxy, butynyloxy, propynylamino, butynylamino, propynyloxy-carbonyle ou butynyloxycarbonyle, chacun éventuellement substitué par du fluor ou du chlore, un reste cyclopropyle, cyclobutyle, cyclopentyle, cyclohexyle, cyclopropyloxy, cyclobutyloxy, cyclopentyloxy, cyclohexyloxy, cyclopropylméthyle, cyclobutylméthyle, cyclopentylméthyle, cyclohexylméthyle, cyclcpropyl-méthoxy, cyclobutylméthoxy, cyclopentylméthoxy, cyclohexylméthoxy, cyclopentylidène-amino, cyclohexylidèneamino, cyclopentyloxycarbonyle, cyclohexyloxycarbonyle, cyclopentylméthoxycarbonyle ou cyclohexylméthoxycarbonyle chacun éventuellement substitué par un radical fluoro, chloro, cyano, carboxy, méthyle, éthyle, n-propyle, isopropyle, méthoxycarbonyle ou éthoxycarbonyle, ou un reste phényle, phényloxy, benzyle, phényléthyle, benzyloxy, phényloxycarbonyle, benzyloxycarbonyle, un reste (éventuellement hydrogéné en totalité ou en partie dans chaque cas) pyrrolyle, pyrazolyle, imidazolyle, thiazolyle, furyle, thiényle, oxazolyle, isoxazolyle, thiazolyle, isothiazolyle, triazinyle, pyrazolylméthyle, furylméthyle, thiénylméthyle, oxazolylméthyle, isoxazolméthyle, thiazoleméthyle, pyridinylméthyle, pyrimidinylméthyle, pyrazolylméthoxy, furylméthoxy ou pyridylméthoxy chacun éventuellement substitué par un radical nitro, cyano, carboxy, fluoro, chloro, bromo, méthyle, éthyle, n-propyle, isopropyle, trifluoro-méthyle, méthoxy, éthoxy, n-propoxy, isopropoxy, difluorométhoxy, trifluorométhoxy, méthoxycarbonyle et/ou éthoxycarbonyle,
R³ représente l'hydrogène, le fluor ou le chlore.

3. Composé suivant la revendication 1, **caractérisé en ce que**
R¹ représente le fluor,
R² est un groupe -NH-SO₂-C₂H₅,
R³ représente l'hydrogène,
Q¹ est un groupe -CO-,
R⁶ est un reste trifluorométhyle, et
R⁷ est un reste méthyle.

4. Procédé de production d'amides d'acides thiocarboxyliques aromatiques substitués suivant l'une des revendications 1 à 3, **caractérisé en ce qu'**on fait réagir des nitriles aromatiques substitués de formule générale (II) 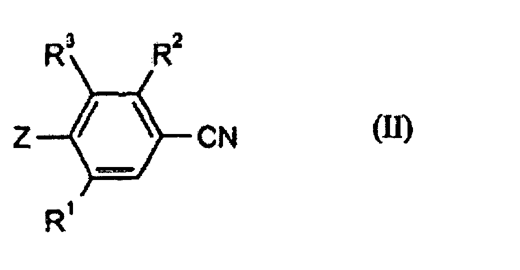 dans laquelle
R¹, R², R³ et Z ont les définitions indiquées dans les revendications 1 à 3,
R¹, R², R³ et Z ont les définitions indiquées dans les revendications 1 à 3,
avec l'hydrogène sulfuré (sulfure d'hydrogène, H₂S) ou le thioacétamide, éventuellement en présence d'un auxiliaire de réaction et en présence éventuelle d'un diluant.

5. Procédé pour combattre des plantes indésirables, **caractérisé en ce qu'**on fait agir des amides d'acides thiocarboxyliques aromatiques substitués suivant l'une des revendications 1 à 3 sur des plantes indésirables et/ou sur leur milieu.

6. Utilisation d'amides d'acides thiocarboxyliques aromatiques substitués suivant l'une des revendications 1 à 3 pour combattre des plantes indésirables.

7. Procédé de préparation de compositions herbicides, **caractérisé en ce qu'**on mélange des amides d'acides thiocarboxyliques aromatiques substitués suivant l'une des revendications 1 à 3 avec des diluants et/ou des substances tensioactives.

8. Compositions herbicides, **caractérisées par** une teneur en au moins un amide d'acide thiocarboxylique aromatique substitué suivant l'une des revendications 1 à 3.
